# EUROPÄISCHE PATENTSCHRIFT

(11) **EP 1 372 459 B1**
(45) Veröffentlichungstag und Bekanntmachung des Hinweises auf die Patenterteilung: **16.12.2009**
(21) Anmeldenummer: 02724263.5
(22) Anmeldetag: 28.03.2002
(51) Int. Cl.: A61B 1/00

(54) **ENDOSKOPISCHE VISUALISIERUNGSVORRICHTUNG MIT UNTERSCHIEDLICHEN BILDSYSTEMEN**
ENDOSCOPIC VISUALISATION DEVICE COMPRISING VARIOUS IMAGE SYSTEMS
DISPOSITIF DE VISUALISATION ENDOSCOPIQUE EQUIPE DE SYSTEMES D'IMAGES DIFFERENTS

(30) Priorität: 30.03.2001 DE 10116056
(43) Veröffentlichungstag der Anmeldung: 02.01.2004
(73) Patentinhaber: Karl Storz GmbH & Co. KG, 78532 Tuttlingen (DE)
(72) Erfinder: IRION, Klaus, M., 78576 Liptingen (DE); STORZ, Karl, Christian, 78532 Tuttlingen (DE)
(74) Vertreter: Heuckeroth, Volker
(86) Internationale Anmeldenummer: PCT/EP2002/003519
(87) Internationale Veröffentlichungsnummer: WO 2002/078528

(56) Entgegenhaltungen:
- WO-A-96/39916
- DE-A- 2 425 827

## Beschreibung

Die Erfindung betrifft eine endoskopische Visualisierungsvorrichtung, mit einem ersten Bildsystem und mit zumindest einem zweiten Bildsystem, wobei von dem ersten Bildsystem ein erstes Bildfeld und von dem zweiten Bildsystem ein zweites Bildfeld erfaßt wird, und wobei die Bildsysteme in einem gemeinsamen Gehäuse angeordnet sind, wobei das erste Bildsystem und das zweite Bildsystem hinsichtlich zumindest einer optischen Kenngröße unterschiedlich sind, und wobei das erste Bildfeld und das zumindest zweite Bildfeld sich nur teilweise überlappen.

Eine derartige endoskopische Visualisierungsvorrichtung ist aus der DE 24 25 827 A bekannt.

Eine endoskopische Visualisierungsvorrichtung der eingangs genannten Art wird beispielsweise zu medizinischen Zwecken bei der sog. minimal-invasiven Chirurgie als Beobachtungssystem verwendet. Bei Standard-Operationen wie beispielsweise der laparoskopischen Cholezystektomie wird üblicherweise über drei kleine künstlich geschaffene Inzisionen in der Bauchdecke der Bauchraum zugängig gemacht. Eine der drei Öffnungen dient der Einführung der Visualisierungsvorrichtung, beispielsweise eines Endoskops, durch die beiden anderen Öffnungen werden die Arbeitsinstrumente, beispielsweise Rohrschaftinstrumente, in den Bauchraum eingeführt. Der chirurgische Eingriff wird durch die vom Chirurgen geführten Instrumente unter visueller Kontrolle über den Monitor ausgeführt, auf dem das von der endoskopischen Visualisierungsvorrichtung gelieferte Bild dargestellt wird. Die endoskopische Visualisierungsvorrichtung selbst wird vom Assistenzpersonal bedient und lagemäßig so gesteuert, daß der Arbeitsbereich der Arbeitsinstrumente sich stets im Bildfeld der Visualisierungsvorrichtung befindet.

Oft besteht der Wunsch, das Operationsgebiet vergrößert oder aus einem anderen Blickwinkel auf dem Monitor darzustellen. Um das Operationsgebiet vergrößert darstellen zu können, ist es bekannt, ein proximalseitig oder distalseitig einstellbares Zoom-Objektiv zu verwenden, oder das Endoskop wird zu diesem Zweck axial verschoben. Um das Operationsgebiet aus einem anderen Blickwinkel darzustellen, ist es erforderlich, nacheinander verschiedene Endoskope mit unterschiedlichen Blickrichtungen zu verwenden. Sowohl die Einstellung eines Zoom-Objektivs, die axiale Verschiebung des Endoskops als auch der Wechsel verschiedener Endoskope stellen zusätzliche Manipulationen und damit einen zusätzlichen Aufwand dar, so daß sich die Operationszeiten verlängern und sich die Kosten von Operationen aufgrund zusätzlicher Visualisierungsvorrichtungen erhöhen.

Im Stand der Technik sind jedoch Anordnungen bekannt, die eine Mehrfachdarstellung des Operationsbereichs und damit eine Abhilfe hinsichtlich der vorstehend genannten Nachteile anbieten.

Aus der DE 38 06 190 A1 ist eine elektronische Endoskopeinrichtung bekannt, die ein längliches Einführteil umfaßt, zwei bildformende optische Systeme in Form von zwei Objektiven am distalen Ende des Einführteils und eine Abbildungsvorrichtung in Form eines elektronischen Bildaufnehmers, der den beiden Objektiven zugeordnet ist. Mit dieser bekannten endoskopischen Visualisierungsvorrichtung ist es möglich, einen Stereoblick zu gewinnen. Diese endoskopische Visualisierungsvorrichtung umfaßt somit zwei Bildsysteme, die jeweils ein Bildfeld erfassen. Die durch die beiden Bildsysteme erfaßten Bildfelder sind jedoch im wesentlichen identisch und überlappen sich im wesentlichen vollständig. Die beiden Objektive der beiden Bildsysteme und damit diese selbst sind zu diesem Zweck hinsichtlich ihrer optischen Eigenschaften identisch. Die beiden Bildsysteme unterscheiden sich lediglich geringfügig in ihren Blickrichtungen, was auch erforderlich ist, damit die beiden nebeneinanderliegenden Bildsysteme dasselbe Bildfeld erfassen, um einen räumlichen Eindruck von diesem Bildfeld zu erhalten.

Eine damit vergleichbare endoskopische Visualisierungsvorrichtung ist aus der DE 42 41 938 A1 bekannt, die ein Endoskop mit Stereo-Seitblickoptik beschreibt. Auch bei diesem bekannten Visualisierungssystem überlappen sich die Bildfelder der beiden Bildsysteme nahezu vollständig und unterscheiden sich die Bildsysteme hinsichtlich ihrer Abbildungseigenschaften nicht, um eben ein Stereobild bzw. einen räumlichen Eindruck von einem beobachteten Objekt zu vermitteln. Außer dieser stereoskopischen Information ist es bei den beiden zuvor genannten bekannten Visualisierungsvorrichtungen nicht möglich, weitere Bildinformationen über das Operationsgebiet zu erhalten.

Ferner ist aus der US 5,166,787 ein Videoendoskop bekannt, das gemäß einem Ausführungsbeispiel zwei Bildsysteme bestehend aus jeweils einem Objektiv und einem elektronischen Bildaufnehmer aufweist. Gemäß einer Variante dieses bekannten Endoskops sind die beiden Bildsysteme in der Lage, einen Stereoskopie-Effekt zu erzeugen, in der die beiden Bildsysteme in eine Lage verschwenkt werden, in der die beiden Bildsysteme zwei Bildfelder erfassen, die sich wiederum nahezu vollständig überlappen. Gemäß einer weiteren Alternative sind zwei Bildsysteme in dem Endoskop vorhanden, deren Blickrichtung sich um 180° unterscheidet, so daß die von den beiden Bildsystemen erfaßten Bildfelder vollständig disjunkt sind. Hierdurch wird zwar ein insgesamt größeres Gesamt-Bildfeld erzielt, jedoch können aus ein und demselben beobachteten Areal keine zusätzlichen Bildinformationen erhalten werden. Davon abgesehen sind bei diesem Endoskop die bereits zuvor genannten unerwünschten Manipulationen erforderlich, um die beiden Bildsysteme in die entsprechende aktive Position zu bewegen.

Eine Vorrichtung gemäß der Präambel des Anspruchs 1 ist aus dem Dokument WO-A-96/39916 bekannt.

Der Erfindung liegt die Aufgabe zugrunde, eine endoskopische Visualisierungsvorrichtung der eingangs genannten Art dahingehend weiterzubilden, daß mit ein und derselben Visualisierungsvorrichtung bei gleichzeitig geringerem Raumbedarf der Vorrichtung ein höherer Gehalt an Bildinformationen erhalten werden kann, ohne daß dazu Manipulationen an der Visualisierungsvorrichtung oder gar ein Wechsel der Visualisierungsvorrichtung erforderlich ist.

Erfindungsgemäß wird diese Aufgabe mittels der Vorrichtung des Anspruchs 1 gelöst.

Die erfindungsgemäße endoskopische Visualisierungsvorrichtung unterscheidet sich von den rein stereoskopischen Visualisierungssystemen dadurch, daß sich das erste Bildfeld und das zumindest zweite Bildfeld nur teilweise überlappen, so daß die Bildfelder unterschiedliche Erfassungsbereiche überdecken. Von der bekannten endoskopischen Visualisierungsvorrichtung, bei der die beiden Bildsysteme vollständig unterschiedliche, d.h. disjunkte Bildfelder erfassen, unterscheidet sich die erfindungsgemäße Visualisierungsvorrichtung dadurch, daß sich die zumindest zwei Bildfelder zumindest teilweise überlappen. In dem teilweisen Überlappungsbereich der von den zumindest zwei Bildsystemen erfaßten Bildfeldern kann nicht nur stereoskopische Bildinformation, sondern darüber hinaus weitere Bildinformation erhalten werden, beispielsweise dann, wenn sich die beiden Bildsysteme hinsichtlich ihres Öffnungswinkels als optische Kenngröße unterscheiden, wie in einer nachfolgend beschriebenen bevorzugten Ausgestaltung vorgesehen ist. Gegenüber den reinen Stereo-Visualisierungssystemen wird durch die nur teilweise Überlappung der zumindest zwei Bildfelder ein insgesamt größerer Bildbereich erfaßt, wodurch ebenfalls weitere zusätzliche Bildinformation erhalten werden kann. Mit der erfindungsgemäßen endoskopischen Visualisierungsvorrichtung wird mit anderen Worten ein "multivisuelles" Visualisierungssystem geschaffen, das die Gewinnung zusätzlicher Bildinformationen ohne zusätzliche Manipulationen durch das Assistenzpersonal und ohne erhöhten instrumentellen Aufwand ermöglicht. Während bei stereoendoskopischen Systemen allenfalls ein auf Toleranzen beruhender Unterschied der optischen Kenngrößen der beiden Bildsysteme auftreten kann, sollen sich die Bildsysteme der erfindungsgemäßen Visualisierungsvorrichtung signifikant voneinander hinsichtlich zumindest einer optischen Kenngröße unterscheiden.

Erfindungsgemäß weist das erste Bildsystem einen ersten elektronischen Bildaufnehmer und das zumindest zweite Bildsystem einen zweiten elektronischen Bildaufnehmer auf.

Die vorstehend genannte Maßnahme hat den besonderen Vorteil, daß die gesamte Visualisierungsvorrichtung sehr schmalbauend ausgestaltet werden kann, was für medizinische Anwendungen stets gefordert wird. Um zumindest zwei Bildsysteme auf der Basis optischer Bildübertragungssysteme in einem Standard-Endoskop zu integrieren, müßten die beiden Bildübertragungssysteme nämlich sehr dünn ausgebildet werden, was mit einem entsprechenden Qualitätsverlust der Bildübertragung verbunden ist. Demgegenüber sind derzeit bereits miniaturisierte elektronische Bildaufnehmer mit maximalen Durchmessern unter 3 mm verfügbar, die den Einsatz mehrerer solcher Systeme in einem Endoskop ermöglichen.

Weiterhin führt für den zweiten und ggf. jeden weiteren Bildaufnehmer nur jeweils eine zusätzliche Signalleitung zur Videobildübertragung von distal nach proximal, während die Signale zum Auslesen und zur Spannungsversorgung der Bildaufnehmer für alle Bildaufnehmer gemeinsam genutzt werden.

Bei einer solchen Schaltung der elektronischen Bildaufnehmer ist es möglich, mit nur einer Ansteuerschaltung und der gleichen Anzahl von Zuleitungen für die Spannungsversorgung zum Auslesen und für die Taktsignale der Bildaufnehmer auszukommen wie für einen einzigen elektronischen Bildaufnehmer, wobei dann nur jeweils ein zusätzliches Kabel pro Bildaufnehmer als Signalleitung für das Videooutput-Signal erforderlich ist.

In einer bevorzugten Ausgestaltung unterscheidet sich das erste Bildsystem von dem zumindest zweiten Bildsystem hinsichtlich der Blickrichtung.

Bei dieser Ausgestaltung besteht der Vorteil darin, daß gegenüber den üblichen stereoskopischen Visualisierungseinheiten ein insgesamt größeres Gesamt-Bildfeld erhalten wird, wobei in dem teilweisen Überlappungsbereich stereoskopische, d.h. räumliche Informationen von einem beobachteten Objekt erhalten werden können. Die Blickrichtungen sind auch nicht nur geringfügig unterschiedlich und zueinander konjugiert, wie beispielsweise bei Stereoendoskop-Systemen, sondern unterscheiden sich wiederum signifikant. Im Unterschied zu Stereo-Endoskopen können die Blickrichtungen auch so gewählt sein, daß sie keinen Schnittpunkt im Gesamtbildfeld besitzen, d.h. divergieren, mit der Maßgabe, daß sich die Bildfelder teilweise überlappen.

In einer weiteren bevorzugten Ausgestaltung unterscheidet sich das erste Bildsystem von dem zumindest zweiten Bildsystem hinsichtlich des Öffnungswinkels.

Durch diese Ausgestaltung werden vorteilhafterweise zwei Bildsysteme mit unterschiedlichen Abbildungseigenschaften miteinander in ein und derselben Visualisierungsvorrichtung kombiniert, wobei sich die Abbildungseigenschaften in einer unterschiedlichen Vergrößerung des beobachteten Bildes manifestieren. Ein Zoom-Objektiv, wie es im Stand der Technik vorgesehen ist, und das entsprechend vom Assistenzpersonal eingestellt werden muß, kann somit bei der erfindungsgemäßen Visualisierungsvorrichtung entfallen. Insbesondere im Überlappungsbereich der beiden Bildfelder tritt zu der dort erhaltenen quasi-stereoskopischen Bildinformation noch eine weitere Bildinformation hinzu, die sich durch unterschiedliche Zoom-Faktoren ergibt. Auf diese Weise läßt sich die erfindungsgemäße Visualisierungsvorrichtung vorteilhafterweise zur Vermessung von Objekten und zur Abstandsmessung zwischen der Visualisierungsvorrichtung und einem Objekt, beispielsweise Gewebe im menschlichen Körper, nutzen.

Selbstverständlich läßt sich die zuvor genannte Ausgestaltung, wonach sich das erste Bildsystem von dem zumindest zweiten Bildsystem hinsichtlich des Öffnungswinkels unterscheidet, auch mit der davor genannten Ausgestaltung kombinieren, wonach sich das erste Bildsystem von dem zumindest zweiten Bildsystem hinsichtlich der Blickrichtung unterscheidet. Der Informationsgewinn des erhaltenen Bildes wird somit noch weiter erhöht. Darüber hinaus versteht es sich, daß nicht nur zwei unterschiedliche Bildsysteme, sondern auch drei und mehr unterschiedliche Bildsysteme in der zuvor genannten Weise in ein und derselben Visualisierungsvorrichtung integriert werden können, wenn dies aus Platzgründen möglich ist. Hierbei ist zu berücksichtigen, daß endoskopische Visualisierungsvorrichtungen zur Verwendung in der minimal-invasiven Chirurgie besondere Anforderungen an die maximale Baugröße erfüllen sollten.

In einer weiteren bevorzugten Ausgestaltung weist das erste Bildsystem ein dem ersten Bildaufnehmer zugeordnetes erstes Objektiv und das zumindest zweite Bildsystem ein dem zweiten Bildaufnehmer zugeordnetes zweites Objektiv auf, und unterscheidet sich das erste Objektiv von dem zweiten Objektiv signifikant hinsichtlich der zumindest einen optischen Kenngröße.

Um eine Bildaufrichtung des beobachteten Bilds zu ermöglichen, ist der erste Bildaufnehmer und/oder der zweite Bildaufnehmer bevorzugt um eine Achse quer zur Bildaufnahmefläche drehbar.

Eine Bildaufrichtung kann jedoch auch über eine entsprechende Datenverarbeitung in der Bildverarbeitungseinheit, der die Videooutput-Signale zugespeist werden, realisiert werden.

In einer weiteren bevorzugten Ausgestaltung ist den Bildsystemen zumindest ein Beleuchtungssystem zugeordnet, das Licht so abstrahlt, daß jedes Bildfeld ausgeleuchtet ist.

Diese Maßnahme hat den Vorteil, daß alle von den Bildsystemen erfaßten Bildfelder entsprechend der zuvor genannten Ausgestaltungen ausreichend ausgeleuchtet sind und gewährleistet ist, daß alle zusätzlich gewonnenen Bildinformationen verwertet werden.

In einer weiteren bevorzugten Ausgestaltung ist eine Positioniereinrichtung zum automatischen Nachführen der Visualisierungsvorrichtung in Abhängigkeit von einer Position eines Arbeitsinstruments vorgesehen, wobei die Positioniereinrichtung so wirkt, daß das Arbeitsinstrument stets in dem einen der Bildfelder erscheint.

Besonders vorteilhaft ist diese Ausgestaltung im Zusammenhang mit der oben genannten Ausgestaltung, wonach sich die zumindest zwei Bildsysteme hinsichtlich ihres Öffnungswinkels unterscheiden. Die Positioniereinrichtung kann beispielsweise so ausgebildet sein, daß eine Nachführung der Visualisierungsvorrichtung dann erfolgt, wenn sich das Arbeitsinstrument aus dem gezoomten Bild des einen Bildsystems mit kleinerem Öffnungswinkel heraus in das Übersichtsbild des Bildsystems mit größerem Öffnungswinkel bewegt, so daß das Arbeitsinstrument stets in dem Bildfeld des Bildsystems kleineren Öffnungswinkels erscheint.

Das Bild größeren Öffnungswinkels kann dem Chirurgen als Übersichtsbild zur besseren Orientierung im Operationsraum dienen, während das Bildfeld kleineren Öffnungswinkels und damit vergrößertem Bild eine höher auflösende Beobachtung der Spitze des Arbeitsinstruments und des in deren Nähe befindlichen Gewebes ermöglicht.

Die erfindungsgemäße Visualisierungsvorrichtung kann in einer bevorzugten Ausgestaltung in Form eines Endoskops ausgebildet sein, wobei die zumindest zwei Bildsysteme in einem distalen Ende eines Schafts des Endoskops angeordnet sind. Insbesondere in Verbindung der Ausgestaltung der Bildsysteme mit elektronischen Bildaufnehmern kann der Schaft vorteilhafterweise sehr schmalbauend ausgebildet werden.

Alternativ hierzu ist es ebenso bevorzugt, wenn die endoskopische Visualisierungsvorrichtung in Form einer Videokameraeinheit, die die zumindest zwei Bildsysteme gemäß einer oder mehreren der zuvor genannten Ausgestaltungen aufweist, ausgebildet ist, die an einem Führungsschaft zum Führen eines Arbeitsinstruments befestigt ist.

Durch diese Ausgestaltung läßt sich die Anzahl von in der Körperoberfläche zu schaffenden Inzisionen bei einer minimal-invasiven Operation verringern, wenn die endoskopische Visualisierungsvorrichtung, an dem Führungsschaft befestigt ist, durch den das Arbeitsinstrument in das Operationsgebiet eingeführt ist. Auch kann hierdurch eine sehr einfache, insbesondere mechanisch wirkende Positioniereinrichtung zum Nachführen der Visualisierungsvorrichtung in Abhängigkeit von der Position des Arbeitsinstruments realisiert werden.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hier noch näher beschrieben. Es zeigen:
- Fig. 1: eine endoskopische Visualisierungsvorrichtung in einer Gesamtdarstellung und ihre Verbindung mit Peripheriegeräten;
- Fig. 2: einen Längsschnitt durch das distale Ende der endoskopischen Visualisierungsvorrichtung in Fig. 1 in einem gegenüber Fig. 1 vergrößerten Maßstab;
- Fig. 3: eine Vorderansicht des distalen Endes der endoskopischen Visualisierungsvorrichtung in Fig. 2;
- Fig. 4: einen Längsschnitt durch ein distales Ende einer endoskopischen Visualisierungsvorrichtung in einem gegenüber Fig. 2 und 3 abgewandelten Gerät, das Teil der Erfindung ist. ; und
- Fig. 5: einen Schnitt entlang der Linie V-V in Fig. 4.

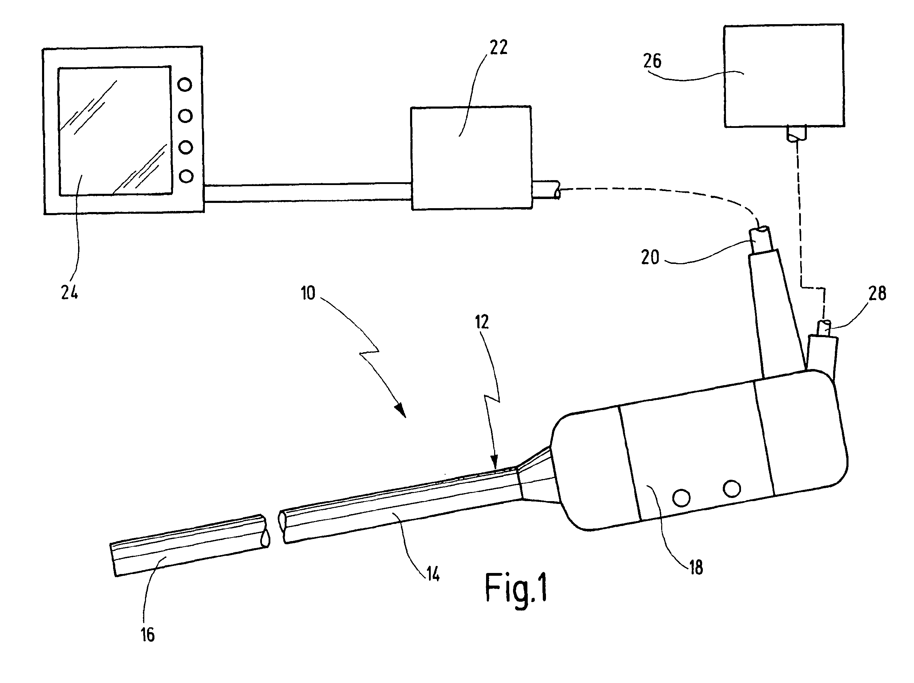

In Fig. 1 ist eine mit dem allgemeinen Bezugszeichen 10 versehene endoskopische Visualisierungsvorrichtung dargestellt. Die endoskopische Visualisierungsvorrichtung 10 ist in dem gezeigten Ausführungsbeispiel in Form eines Endoskops 12 ausgebildet.

Die endoskopische Visualisierungsvorrichtung 10 in Form des Endoskops 12 wird im Rahmen der minimal-invasiven Chirurgie verwendet.

Das Endoskop 12 weist einen langerstreckten Schaft auf, dessen distaler Endabschnitt bzw. distales Ende mit dem Bezugszeichen 16 versehen ist.

In dem gezeigten Ausführungsbeispiel ist der Schaft 14 insgesamt starr ausgebildet, jedoch läßt sich die vorliegende Erfindung genauso gut bei einem flexiblen Endoskop mit einem entsprechend flexiblen Schaft einsetzen.

Das Endoskop 12 ist ferner als Video-Endoskop ausgebildet und weist daher am proximalen Ende des Schafts 14 kein Okular, sondern ein Handstück 18 auf. Von dem Handstück 18 führt ein Kabel 20 zur elektrischen Signalübertragung zu einer Bildverarbeitungseinheit 22, die wiederum mit einem Monitor 24 zur Darstellung endoskopischer Bilder, die von dem Endoskop 12 geliefert werden, verbunden ist.

Des weiteren ist eine Lichtquelle 26 vorgesehen, die Licht erzeugt, das in das Endoskop 12 durch das Handstück 18 hindurch in den Schaft 14 bis zum distalen Ende 16 eingespeist wird. Die Lichtquelle 26 ist über ein Lichtleitkabel 28 entsprechend mit dem Handstück 18 des Endoskops 12 verbunden.

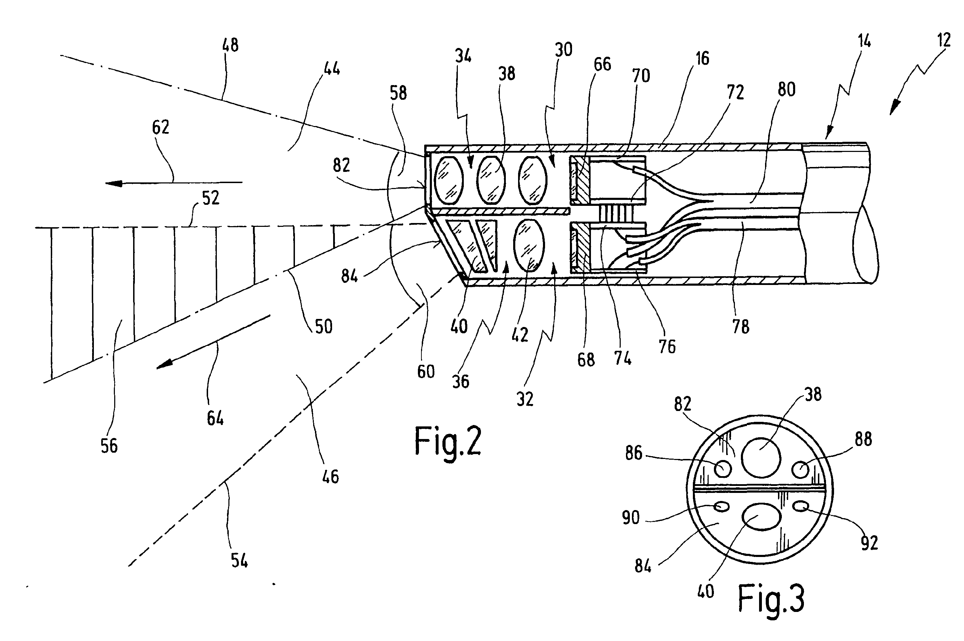

In Fig. 2 und 3 ist der distale Abschnitt 16 des Schafts 14 des Endoskops 12 im vergrößerten Maßstab mit weiteren Einzelheiten dargestellt.

In dem Schaft 14 ist ein erstes Bildsystem 30 und parallel dazu ein zweites Bildsystem 32 angeordnet.

Das erste Bildsystem 30 weist ein erstes Objektiv 34 auf, während das zweite Bildsystem 32 ein zweites Objektiv 36 aufweist. Das erste Objektiv 34 umfaßt eine hier nur beispielhaft und symbolisch dargestellte Anordnung aus Linsen 38 auf, während das zweite Objektiv 36 eine Anordnung, die hier ebenfalls nur beispielhaft zu verstehen ist, aus zwei Prismen 40 und einer Linse 42 aufweist.

Die symbolische Darstellung der Objektive 34 und 36 soll hier veranschaulichen, daß die beiden Objektive 36 und 38 unterschiedliche optische Kenngrößen aufweisen.

Die optischen Kenngrößen eines endoskopischen Visualisierungssystems bestimmen das von dem entsprechenden Bildsystem erfaßte Bildfeld und die daraus erhaltene Bildinformation. Das erfaßte Bildfeld einschließlich der daraus erhaltenen Bildinformation wird bei einem endoskopischen Visualisierungssystem durch die Blickrichtung als der ersten optischen Kenngröße und durch den Öffnungswinkel als zweiter optischer Kenngröße bestimmt.

Ein von dem ersten Bildsystem 30 erfaßtes Bildfeld ist mit dem Bezugszeichen 44 versehen, während ein von dem zweiten Bildsystem 32 erfaßtes Bildfeld mit dem Bezugszeichen 46 versehen ist. Die Begrenzung des ersten Bildfeldes 44 ist mit strichpunktierten Linien 48 und 50 veranschaulicht, während die Begrenzung des zweiten Bildfelds 46 durch unterbrochene Linien 52 und 54 veranschaulicht ist.

Das erste Bildsystem 30 und das zweite Bildsystem 32, d.h. genauer gesagt das erste Objektiv 34 und das zweite Objektiv 36 sind nun so ausgebildet bzw. orientiert, daß sich das erste Bildfeld 44 und das zweite Bildfeld 46 nur teilweise überlappen, wobei ein entsprechender Überlappungsbereich mit dem Bezugszeichen 56 versehen ist (schraffiert in Fig. 2).

Ein Öffnungswinkel des ersten Bildsystems 30 ist mit dem Bezugszeichen 58 versehen, während ein Öffnungswinkel des zweiten Bildsystems 32 mit dem Bezugszeichen 60 versehen ist. Eine Blickrichtung des ersten Bildsystems 30 ist durch einen Pfeil 62 veranschaulicht, und eine Blickrichtung des zweiten Bildsystems 32 mit einem Pfeil 64.

In dem in Fig. 2 und 3 dargestellten Ausführungsbeispiel des Endoskops 12 in Fig. 1 unterscheiden sich das erste Bildsystem 30 und das zweite Bildsystem 32 hinsichtlich der Blickrichtungen 62 und 64. Das erste Bildsystem 30 weist eine Blickrichtung von etwa 0° bezogen auf die Längsachse des Schafts 14 auf, während die Blickrichtung 64 des zweiten Bildsystems 32 mit der Längsachse des Schafts 14 einen Winkel von etwa 30° einschließt. Die Blickrichtungen 62 und 64 divergieren in diesem Ausführungsbeispiel. Die Öffnungswinkel 58 und 60 unterscheiden sich in dem in Fig. 2 und 3 dargestellten Ausführungsbeispiel hingegen nicht. Außer den zuvor genannten Blickrichtungen sind im Rahmen der Erfindung auch andere Blickrichtungen verwendbar, beispielsweise 0° und 60°, 30° und 60°, 60° und 90°, usw., solange sich die zugeordneten Bildfelder zumindest teilweise, aber nicht vollständig überlappen und die Blickrichtungen signifikant unterschiedlich sind.

Das Endoskop 12 integriert somit in einem einzigen Endoskop zwei Bildsysteme mit unterschiedlichen Blickrichtungen und gleichem Öffnungswinkel. Aus dem Überlappungsbereich 56 der beiden Bildfelder 44 und 46 kann stereoskopische Bildinformation eines sich darin befindlichen Objekts gewonnen werden, d.h. dieses Objekt kann im Überlappungsbereich 56 räumlich gesehen werden. Das gesamte Bild der beiden Bildsysteme 30 und 32, das durch die Linien 48 und 54 in Fig. 2 begrenzt ist, ist jedoch gegenüber einem herkömmlichen Stereo-Endoskop, bei dem sich beide Bildfelder vollständig überlagern, wesentlich vergrößert.

Das erste Bildsystem 30 und das zweite Bildsystem 32 weisen ferner jeweils einen elektronischen Bildaufnehmer 66 bzw. 68 auf, auf die das jeweilige Objektiv 34 bzw. 36 abbildet. Die unmittelbare Zuordnung der Bildaufnehmer 66 und 68 zu den Objektiven 34 und 36, d.h. die distalseitige Anordnung der Bildaufnehmer 66 und 68 hat den Vorteil, daß optische Übertragungssysteme wie Lichtleiter oder Relaislinsensysteme, die einen entsprechenden Durchmesser benötigen, um eine ausreichende Übertragungsqualität zu gewährleisten, nicht benötigt werden. Jedoch ist es im Rahmen der vorliegenden Erfindung ebenso möglich, die Bildaufnehmer 66 und 68 am proximalen Ende des Endoskops, beispielsweise im Handstück 18, anzuordnen und ausgehend von den Objektiven 34 bzw. 36 eine Lichtübertragung durch Lichtleiter und dgl. zu den Bildaufnehmern vorzusehen.

Die Bildaufnehmer 66 und 68 sind in Verbindung mit einem Tape Automated Bonding (TAB)-Package ausgeführt, bei denen die Anschlußpads 70 und 72 des ersten Bildaufnehmers 66 und die Anschlußpads 74 und 76 des zweiten Bildaufnehmers 68 spiegelbildlich zueinander belegt sind. Die Pads 72 und 74 sind direkt miteinander kontaktiert, wie in Fig. 2 dargestellt ist. Dadurch kann die Ansteuerung der beiden Bildaufnehmer 66 und 68 über eine gemeinsame Zuleitung erfolgen, während lediglich die Videooutput-Signale getrennt verlaufen und entsprechend an den Pads 70 und 76 abgegriffen werden. Entsprechende elektrische Leitungen sind mit dem Bezugszeichen 78 und 80 versehen.

Zumindest einer der Bildaufnehmer 66 und 68 ist um eine parallel zur Längsmittelachse des Schafts 14 verlaufenden Achse, die quer zur frontalen Bildaufnahmefläche des entsprechenden Bildaufnehmers 66 bzw. 68 verläuft, drehbar, um eines der beiden den Bildfeldern 44 bzw. 46 entsprechenden Bildern aufzurichten bzw. dem anderen Bildfeld entsprechend anzupassen. Anstelle der Drehbarkeit der Bildaufnehmer 66 und/oder 68 kann auch eine entsprechende Bildaufrichtung in der Bildverarbeitungseinheit 22 erfolgen.

Lichteintrittsseitige Flächen 82 und 84 der Bildsysteme 30 und 32 sind entsprechend der unterschiedlichen Blickrichtungen 62 und 64 um einen Winkel von etwa 30° gegeneinander geneigt. Jedem der Bildsysteme 30 und 32 ist ein entsprechendes Beleuchtungssystem zugeordnet, wobei das Beleuchtungssystem einen ersten Lichtleiter 86 und einen zweiten Lichtleiter 88 umfaßt, deren lichtaustrittsseitiges Ende in der Fläche 82 liegt, so daß die Lichtleiter 86 und 88 von der Lichtquelle 26 erzeugtes Licht in das Bildfeld 44 abstrahlen. Entsprechend weist das Beleuchtungssystem zwei weitere Lichtleiter 90 und 92 auf, die in der Fläche 84 münden und dementsprechend so gerichtet sind, daß sie das Bildfeld 46 vollständig ausleuchten.

Anstelle von einem oder mehreren Lichtleitern wie in Fig. 3 können an entsprechender Stelle auch Lichtquellen wie Leuchtdioden angeordnet sein, die entsprechend über eine elektrische Zuleitung von proximal gespeist werden.

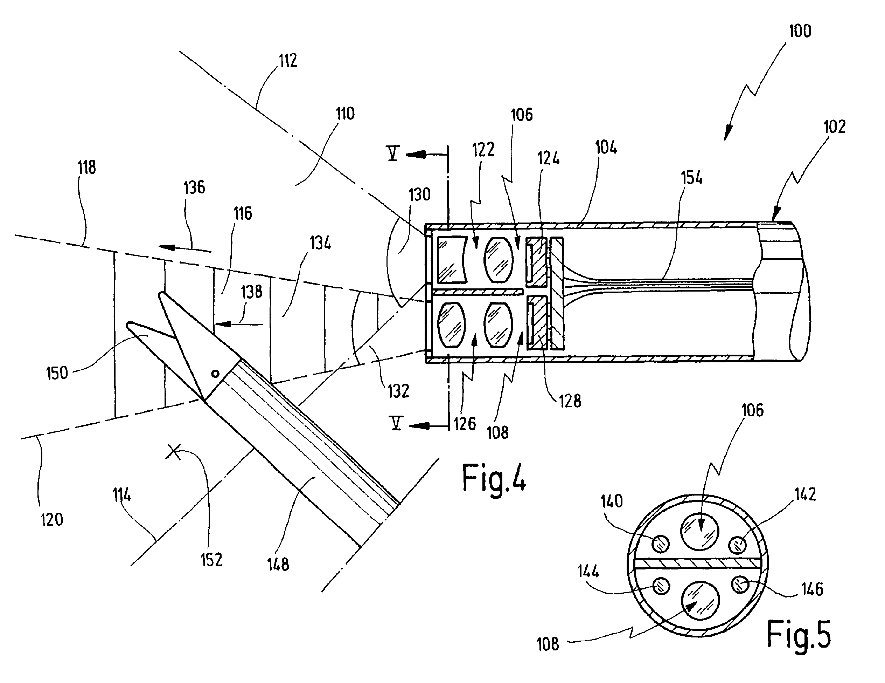

In Fig. 4 und 5 ist eine endoskopische Visualisierungsvorrichtung dargestellt, die nicht Teil der Erfindung ist, wobei diese endoskopische Visualisierungsvorrichtung wiederum in Form eines Endoskops 100 vergleichbar dem Endoskop 12 ausgebildet ist. Das Endoskop 100 weist einen Schaft 102 auf, in dessen distalem Abschnitt 104 ein erstes Bildsystem 106 und ein zweites Bildsystem 108 angeordnet sind.

Das erste Bildsystem 106 erfaßt ein erstes Bildfeld 110, dessen Grenzen durch Linien 112 und 114 veranschaulicht sind, während das zweite Bildsystem 108 ein zweites Bildfeld 116 erfaßt, dessen Begrenzung durch Linien 118 bzw. 120 veranschaulicht ist.

Das erste Bildsystem 106 weist ein erstes Objektiv 122 und einen ersten elektronischen Bildaufnehmer 124, und das zweite Bildsystem 108 weist ein zweites Objektiv 126 und einen zweiten Bildaufnehmer 128 auf.

Das erste Bildsystem 106 und das zweite Bildsystem 108 unterscheiden sich wiederum hinsichtlich zumindest einer optischen Kenngröße, und zwar im vorliegenden Fall hinsichtlich ihres Öffnungswinkels 130 bzw. 132. Der Öffnungswinkel 130 des ersten Bildsystems 106, der durch das erste Objektiv 122 vorgegeben ist, ist dabei größer als der Öffnungswinkel 132 des zweiten Bildsystems 108, der durch dessen Objektiv 126 bestimmt wird.

Die entsprechenden von dem Bildsystem 106 und 108 erfaßten Bildfelder 110 und 116 überlappen sich wie im vorhergehenden Ausführungsbeispiel nur teilweise. Ein entsprechender Überlappungsbereich ist in Fig. 4 schraffiert dargestellt und mit dem Bezugszeichen 134 versehen.

Ein im Überlappungsbereich 134 befindliches Objekt wird durch das zweite Bildsystem 108 mit dem kleineren Öffnungswinkel 132 vergrößert gesehen, so daß das zweite Bildsystem 108 einem Zoom-Objektiv gleichkommt, während das gleiche Objekt im Überlappungsbereich 134 gleichzeitig im Bildfeld 110 des ersten Bildsystems 106 mit größerem Öffnungswinkel erscheint, wodurch diese Bildinformation dazu genutzt werden kann, die Lage des Objekts bezüglich der Umgebung im Operationsbereich im Sinne eines Übersichtsbildes zu erkennen.

Bei dieser Ausgestaltung ist es somit mit nur dem einen Endoskop 100 möglich, von ein und demselben Objekt einerseits ein Übersichtsbild (Totale), andererseits ein vergrößertes Bild zu erhalten. Darüber hinaus ergeben sich im Überlappungsbereich 134 der Bildfelder 110 und 116 quasi-stereoskopische Effekte, die eine räumliche exakte Geometrieerfassung des beobachteten Objekts und auch eine Abstandsmessung zwischen dem distalen Ende 104 des Endoskops 100 und dem Objekt zu erhalten.

Der Öffnungswinkel 130 des ersten Bildsystems 106 beträgt beispielsweise 75°, und der zweite Öffnungswinkel 132 des zweiten Bildsystems 108 beispielsweise 40°.

Während die Objektive 122 und 126 in Fig. 4 eine Blickrichtung von etwa 0° zur Längsachse des Schafts 102 aufweisen, wie mit Pfeilen 136 und 138 angedeutet ist, können jedoch auch entsprechende Objektive mit dem gleichen Öffnungswinkel, jedoch unterschiedlichen Blickrichtungen, vorgesehen werden.

Das Gerät gemäß Fig. 4 kann mit Teilen des Ausführungsbeispiels gemäß Fig. 2 kombiniert werden, d.h. es können zwei oder mehr Bildsysteme mit sowohl unterd. h. es können zwei oder mehr Bildsysteme mit sowohl unterschiedlichen Öffnungswinkeln als auch unterschiedlichen Blickrichtungen in ein und derselben Visualisierungsvorrichtung miteinander kombiniert werden.

Gemäß Fig. 5 ist den Bildsystemen 106 und 108 wiederum zumindest ein Beleuchtungssystem in Form von Lichtleitern 140 bis 146 zugeordnet, das eine entsprechende Ausleuchtung der Bildfelder 110 und 116 gewährleistet. Um dem größeren Öffnungswinkel 130 des Bildsystems 106 entsprechend eine ausreichende Ausleuchtung des Bildfelds 110 zu gewährleisten, sind den Lichtleitern 140 und 142 beispielsweise entsprechende Aufweitungsoptiken zugeordnet.

In Fig. 4 ist des weiteren ein Arbeitsinstrument 148 dargestellt, dessen distale Spitze 150 gleichzeitig im Bildfeld 116 des zweiten Bildsystems 108 und gleichzeitig im ersten Bildfeld 110 des ersten Bildsystems 106, mit anderen Worten im Überlappungsbereich 134 beider Bildfelder 110 und 116 liegt.

Es ist eine nicht dargestellte Positioniereinrichtung zum automatischen Nachführen der Visualisierungsvorrichtung bzw. des Endoskops 100 vorgesehen, die das Endoskop 100 in Abhängigkeit von der Position des Arbeitsinstruments 148, beispielsweise in Abhängigkeit von der Position der Spitze 150 des Arbeitsinstruments 148 derart nachführt, daß die Spitze 150 des Arbeitsinstruments 148 stets in dem zweiten Bildfeld 116 erscheint, in dem die Spitze 150 des Arbeitsinstruments 148 vergrößert abgebildet wird.

Bewegt sich bei einer Manipulation des Arbeitsinstruments 148 bei einem chirurgischen Eingriff derart, daß die Spitze 150 außerhalb des Bildfelds 116 zu liegen kommt, beispielsweise an einer mit dem Bezugszeichen 152 versehenen Stelle, wird die Positioniereinrichtung aktiv und führt das Endoskop 100 so nach, daß die Spitze 150 wieder in dem Bildfeld 116, d.h. vergrößert erscheint. Solange die Spitze 150 im Bildfeld 116 erscheint, ist die Positioniereinrichtung inaktiv, d.h. eine Bewegung der Spitze 150 im Bildfeld 116 führt vorzugsweise nicht zu einer Nachführung des Endoskops 100. Die Positioniereinrichtung weist entsprechende Positionssensoren auf, um die Lage der Spitze 150 des Arbeitsinstruments 148 entsprechend zu detektieren. Die Positioniereinrichtung kann entsprechend durch die Bildverarbeitungseinheit gesteuert werden.

Ferner ist in Fig. 4 dargestellt, daß die Bildaufnehmer 124 und 128 auf einem gemeinsamen Pad, d.h. einer gemeinsamen Platine kontaktiert sind, wobei für beide Bildaufnehmer 124 und 128 nur eine Zuleitung zur Speisung der Bildaufnehmer erforderlich ist, während die Videooutput-Signale entsprechend getrennt nach proximal zur Bildverarbeitungseinheit 22 geführt werden müssen. Entsprechende Leitungen 154 sind in Fig. 4 dargestellt.

Des weiteren können auch mehr als zwei Bildsysteme in einer endoskopischen Visualisierungsvorrichtung integriert sein, wodurch der gewonnene Informationsgehalt der endoskopischen Bilder noch weiter erhöht werden kann.

Während in den Ausführungsbeispielen die endoskopische Visualisierungsvorrichtung jeweils in Form eines Endoskops ausgebildet ist, kann die endoskopische Visualisierungsvorrichtung jedoch auch eine Videokameraeinheit aufweisen, die die zumindest zwei Bildsysteme aufweist, und die an einem Führungsschaft zum Führen eines Arbeitsinstruments, wie demjenigen des Arbeitsinstruments 148 in Fig. 4, befestigt ist. Auf diese Weise kann eine mechanische Kopplung zwischen der endoskopischen Visualisierungsvorrichtung und dem Arbeitsinstrument erreicht werden, die eine einfache Art der automatischen Nachführung der endoskopischen Visualisierungsvorrichtung in Abhängigkeit der Position des Arbeitsinstruments ermöglicht. Eine solche Videokameraeinheit mit zumindest zwei Bildsystemen kann im Prinzip dem Aufbau der distalen Enden 16 bzw. 104 der Endoskope 12 bzw. 100 in ebenso miniaturisierter Ausführung entsprechen.

## Patentansprüche

1. Endoskopische Visualisierungsvorrichtung, mit einem ersten Bildsystem (30) und mit zumindest einem zweiten Bildsystem (32), wobei von dem ersten Bildsystem (30) ein erstes Bildfeld (44) und von dem zweiten Bildsystem (32) ein zweites Bildfeld (46) erfaßt wird, und wobei die Bildsysteme (30, 32) in einem gemeinsamen Gehäuse angeordnet sind, wobei das erste Bildsystem (30) und das zweite Bildsystem (32) hinsichtlich zumindest einer optischen Kenngröße unterschiedlich sind, und wobei das erste Bildfeld (44) und das zumindest zweite Bildfeld (46) sich nur teilweise überlappen, wobei das erste Bildsystem (30) einen ersten elektronischen Bildaufnehmer (66) und das zumindest zweite Bildsystem einen zweiten elektronischen Bildaufnehmer (68) aufweist, wobei jeder der Bildaufnehmer (66, 68) mit eigenen Anschlußpads (70, 72, 74, 76) versehen ist,
**dadurch gekennzeichnet, daß** die Zuleitung zur Ansteuerung der Bildaufnehmer (66, 68) von den Bildaufnehmern (66, 68) gemeinsam genutzt wird, wobei für den zweiten Bildaufnehmer (68) nur eine zusätzliche Signalleitung zur Videobildübertragung von distal nach proximal führt, und wobei die Anschlußpads (70, 72, 74, 76) des ersten und des zweiten Bildaufnehmers (66, 68) spiegelbildlich belegt und die einander zugewandten Anschlußpads (72, 74) miteinander kontaktiert sind.

2. Visualisierungsvorrichtung nach Anspruch 1, **dadurch gekennzeichnet, daß** sich das erste Bildsystem (30) von dem zumindest einen zweiten Bildsystem (32) hinsichtlich der Blickrichtung (62, 64) unterscheidet.

3. Visualisierungsvorrichtung nach Anspruch 1 oder 2, **dadurch gekennzeichnet, daß** sich das erste Bildsystem von dem zumindest einen zweiten Bildsystem hinsichtlich des Öffnungswinkels unterscheidet.

4. Visualisierungsvorrichtung nach einem der Ansprüche 1 bis 3, **dadurch gekennzeichnet, daß** das erste Bildsystem (30) ein dem ersten Bildaufnehmer (66) zugeordnetes erstes Objektiv (34) und das zumindest zweite Bildsystem (32) ein dem zweiten Bildaufnehmer (68) zugeordnetes zweites Objektiv (36) aufweist, und daß sich das erste Objektiv (34) von dem zweiten Objektiv (36) hinsichtlich der zumindest einen optischen Kenngröße unterscheidet.

5. Visualisierungsvorrichtung.nach einem der Ansprüche 1 bis 4, **dadurch gekennzeichnet, daß** der erste Bildaufnehmer (66) und/oder der zweite Bildaufnehmer (68) um eine Achse senkrecht zur Bildaufnahmefläche drehbar ist.

6. Visualisierungsvorrichtung nach einem der Ansprüche 1 bis 5, **dadurch gekennzeichnet, daß** den Bildsystemen (30, 32) zumindest ein Beleuchtungssystem zugeordnet ist, das Licht so abstrahlt, daß jedes Bildfeld (44, 46) ausgeleuchtet ist.

7. Visualisierungsvorrichtung nach einem der Ansprüche 1 bis 6, **gekennzeichnet durch** eine Positioniereinrichtung zum automatischen Nachführen der Visualisierungsvorrichtung in Abhängigkeit von einer Position eines Arbeitsinstruments , wobei die Positioniereinrichtung so wirkt, daß das Arbeitsinstrument stets in dem einen der Bildfelder erscheint.

8. Visualisierungsvorrichtung nach einem der Ansprüche 1 bis 7, **dadurch gekennzeichnet, daß** sie in Form eines Endoskops (12) ausgebildet ist, wobei die zumindest zwei Bildsysteme (30, 32) in einem distalen Ende eines Schafts (14) des Endoskops (12) angeordnet sind.

9. Visualisierungsvorrichtung nach einem der Ansprüche 1 bis 7, **dadurch gekennzeichnet, daß** sie in Form einer Videokameraeinheit, die die zumindest zwei Bildsysteme aufweist, ausgebildet ist, die an einem Führungsschaft zum Führen eines Arbeitsinstruments befestigt ist.

## Claims

1. An endoscopic visualization apparatus, comprising a first imaging system (30) and at least one second imaging system (32), a first image field (44) being covered by the first imaging system (30), and a second image field (46) being covered by the second imaging system (32), and the imaging systems (30, 32) being arranged in a common housing, wherein the first imaging system (30) and the second imaging system (32) are significantly different with regard to at least one optical parameter, and wherein the first image field (44) and the at least one second image field (46) overlap one another only partially, wherein the first imaging system (30) has a first electronic imager (66), and the at least one second imaging system has a second electronic imager (68), wherein each of the imagers (66, 68) is provided with connecting pads (70, 72, 74, 76) of its own, **characterized in that** the supply lead for driving the imagers (66, 68) are used jointly by the imagers (66, 68), wherein only one additional signal line for video image transmission leads from distally to proximally for the second imager (68), and wherein the connecting pads (70, 72, 74, 76) of the first and the second imagers (66, 68) are configured in mirror-image fashion and the connecting pads (72, 74) facing one another are contacted with one another.

2. The visualization apparatus of claim 1, **characterized in that** the first imaging system (30) differs from the at least one second imaging system (32) with regard to the direction of view (62, 64).

3. The visualization apparatus of claim 1 or 2, **characterized in that** the first imaging system differs from the at least one second imaging system with regard to the aperture angle.

4. The visualization apparatus of anyone of claims 1 through 3, **characterized in that** the first imaging system (30) has a first objective (34) assigned to the first imager (66), and the at least one second imaging system (32) has a second objective (36) assigned to the second imager (68), and the first objective (34) differs from the second objective (36) with regard to the at least one optical parameter.

5. The visualization apparatus of anyone of claims 1 through 4, **characterized in that** the first imager (66) and/or the second imager (68) can be rotated about an axis transverse to the image recording surface.

6. The visualization apparatus of anyone of claims 1 through 5, **characterized in that** the imaging systems (30, 32) is assigned at least one illuminating system which radiates light such that each image field (44, 46) is illuminated.

7. The visualization apparatus of anyone of claims 1 through 6, **characterized by** a positioning device for automatically tracking the visualization apparatus as a function of a position of an operating instrument, the positioning device acting such that the operating instrument always appears in one of the image fields.

8. The visualization apparatus of anyone of claims 1 through 7, **characterized in that** it is designed in the form of an endoscope (12), the at least two imaging systems (30, 32) being arranged in a distal end of a shaft (14) of the endoscope (12).

9. The visualization apparatus of anyone of claims 1 through 9, **characterized in that** it is designed in the form of a video camera unit which has the at least two imaging systems and which is fastened on a guide shaft for guiding an operating instrument.

## Revendications

1. Dispositif de visualisation endoscopique, avec un premier système d'image (30) et avec au moins un second système d'image (32), un premier champ d'image (44) étant détecté par le premier système d'image (30) et un second champ d'image (46) l'étant par le second système d'image (32) et les systèmes d'image (30, 32) étant disposés dans un boîtier commun, le premier système d'image (30) et le second système d'image (32) étant différents pour ce qui est d'au moins une grandeur caractéristique optique et le premier champ d'image (44) et le au moins un second champ d'image (46) ne se superposant que partiellement, le premier système d'image (30) présentant un premier capteur d'images électronique (66) et le au moins un second système d'image (32) présentant un second capteur d'images (68), chacun des capteurs d'images 66, 68) étant pourvu de bornes de connexion propres (70, 72, 74, 76), **caractérisé en ce que** la conduite en vue de la commande des capteurs d'images (66, 68) est utilisée conjointement par les capteurs d'images (66, 68), sachant que, dans le cas du second capteur d'images (68), une conduite de signaux supplémentaire seulement en vue de la transmission d'images vidéo conduit d'un point distal à un point proximal, et sachant que les bornes de connexion (70, 72, 74, 76) du premier et du second capteurs d'images (66, 68) sont disposées dans une orientation en image miroir les unes par rapport aux autres et que les bornes de connexion tournées l'une vers l'autre (72, 74) sont mises en contact l'une avec l'autre.

2. Dispositif de visualisation selon la revendication 1, **caractérisé en ce que** le premier système d'image (30) se différentie du au moins un second système d'image (32) pour ce qui est de la direction de visée (62, 64).

3. Dispositif de visualisation selon la revendication 1 ou 2, **caractérisé en ce que** le premier système d'image (30) se différentie du au moins un second système d'image pour ce qui est de l'angle d'ouverture

4. Dispositif de visualisation selon l'une quelconque des revendications 1 à 3, **caractérisé en ce que** le premier système d'image (30) présente un premier objectif (34) affecté au premier capteur d'images (66) et **en ce que** le au moins un second système d'image (32) présente un second objectif affecté au second capteur d'images (68), et en ce le premier objectif (34) se différentie du second objectif (36) pour ce qui est d'au moins une grandeur caractéristique optique.

5. Dispositif de visualisation selon l'une quelconque des revendications 1 à 4, **caractérisé en ce que** le premier capteur d'images (66) et/ou le second capteur d'images (68) peut subir une rotation autour d'un axe perpendiculairement à la surface de prise de vue.

6. Dispositif de visualisation selon l'une quelconque des revendications 1 à 5, **caractérisé en ce que** l'on affecte aux systèmes d'image (30, 32) tout au moins un système d'illumination, qui émet de la lumière de telle sorte que chaque champ d'image (44, 46) soit illuminé.

7. Dispositif de visualisation selon l'une quelconque des revendications 1 à 6, **caractérisé par** un équipement de positionnement en vue du déplacement automatique du dispositif de visualisation en fonction de la position d'un instrument de travail, l'équipement de positionnement agissant de telle sorte que l'instrument de travail apparaisse toujours dans l'un des champs d'image.

8. Dispositif de visualisation selon l'une quelconque des revendications 1 à 7, **caractérisé en ce qu'**il est réalisé sous la forme d'un endoscope (12), les au moins deux systèmes d'image (30, 32) étant disposés à une extrémité distale d'une tige (14) de l'endoscope (12).

9. Dispositif de visualisation selon l'une quelconque des revendications 1 à 7, **caractérisé en ce qu'**il est réalisé sous la forme d'une unité de caméra vidéo, qui présente les au moins deux systèmes d'image, et qui est fixée à une tige de guidage en vue du guidage d'un instrument de travail.
